# EUROPÄISCHE PATENTSCHRIFT

(11) **EP 2 400 913 B1**
(45) Veröffentlichungstag und Bekanntmachung des Hinweises auf die Patenterteilung: **22.03.2017**
(21) Anmeldenummer: 10705998.2
(22) Anmeldetag: 25.02.2010
(51) Int. Cl.: A61B 17/86, A61B 50/20, A61B 50/30, A61B 17/80

(54) **TRÄGER UND HALTERUNG FÜR CHIRURGISCHE GEGENSTÄNDE**
SUPPORT AND MOUNTING FOR SURGICAL OBJECTS
SUPPORT ET FIXATION POUR DES OBJETS CHIRURGICAUX

(30) Priorität: 26.02.2009 DE 202009002639 U
(43) Veröffentlichungstag der Anmeldung: 04.01.2012
(73) Patentinhaber: Medartis Holding AG, 4057 Basel (CH)
(72) Erfinder: BRAND, Stefan, CH-4053 Basel (CH); THIEL, Dirk, 79219 Staufen (DE)
(74) Vertreter: Hepp Wenger Ryffel AG
(86) Internationale Anmeldenummer: PCT/EP2010/052435
(87) Internationale Veröffentlichungsnummer: WO 2010/097447

(56) Entgegenhaltungen:
- EP-A2- 1 582 169
- EP-A2- 1 972 290
- WO-A2-2009/024189
- DE-A1-102006 036 460
- DE-C- 807 124
- DE-U1-202007 016 144
- US-A1- 2008 016 989

## Beschreibung

Die vorliegende Erfindung betrifft eine Einheit aus einem Träger zur Aufnahme mindestens eines chirurgischen Gegenstandes und einem solchen chirurgischen Gegenstand sowie ein Osteosynthese-Set gemäss den Oberbegriffen der unabhängigen Patentansprüche.

In der Knochenchirurgie wird oftmals eine Vielzahl von Knochenschrauben, Knochennägeln oder ähnlichen chirurgischen Gegenständen benötigt, welche dem Chirurgen bei der Operation nach Bedarf zur Verfügung stehen müssen. Die oftmals geringen Dimensionen der Knochenschrauben, etc. erschweren dabei deren Handhabung sehr. Insbesondere ist es schwierig, kleine Knochenschrauben oder dergleichen mit der Hand oder einem Schraubendreher zu ergreifen.

Aus WO 2005/092231 ist bereits ein Träger mit einigen Merkmalen des unabhängigen Patentanspruchs 1 bekannt, welcher für die Lagerung und Darbietung einer einzelnen Knochenschraube geeignet ist. Die Schraube ist lose in einer Öffnung des Trägers eingesetzt und muss daher mit einer zusätzlichen Abdeckung gegen ein unbeabsichtigtes Herausfallen gesichert werden.

Diese Anordnung weist jedoch vielerlei Nachteile auf. So ist etwa die Schraube bei aufgesetzter Abdeckung nicht sichtbar, so dass beispielsweise nicht unmittel erkennbar ist, ob es sich um eine Torx- oder eine Kreuzschlitzschraube handelt. Ebenso ist eine allfällige Beschriftung mit Längenangaben auf der Oberseite des Trägers bei aufgesetzter Abdeckung nicht sichtbar. Weiterhin erschwert die Abdeckung die Reinigung und Sterilisation des Schraubenkopfes und führt zu einer vergrösserten Bauhöhe. Darüber hinaus muss die Abdeckung vor der Entnahme der Knochenschraube separat abgelegt werden, was einen weiteren Arbeitsschritt darstellt und daher das Hantieren während einer Operation erschwert. Zudem kann die Knochenschraube aus dem Träger herausfallen, wenn die Abdeckung zur Vorbereitung einer Operation bereits entfernt und der die Träger enthaltende Behälter versehentlich verkippt wird. Weiterhin ist es möglich, eine versehentlich entnommene oder aus dem Träger heraus gefallene Knochenschraube wieder zurück in den Träger einzusetzen und beispielsweise versehentlich in einer späteren Operation zu verwenden, was die gesetzlichen Anforderungen nicht erfüllt. Ausserdem kann eine entnommene oder aus dem Träger heraus gefallene Knochenschraube versehentlich in einen falschen Träger eingesetzt werden, was die Identifikation der Knochenschraube erschweren kann.

Aus WO 01/49198 ist ein elastischer Träger bekannt, in welchen eine Knochenschraube einklemmbar ist. Dieser Träger erlaubt die Entnahme der Knochenschraube nur quer zu ihrer Längsachse. Einer oder mehrere solcher Träger können in einem chirurgischen Behälter liegend gelagert werden, so dass die Knochenschraube direkt vertikal nach oben entnommen werden kann; dies führt jedoch zu einem hohen Platzbedarf in der horizontalen Ebene des Behälters. Alternativ könnten die Träger stehend gelagert werden; in diesem Falle müsste jedoch erst der Träger aus dem Behälter entfernt werden, bevor die Knochenschraube entnommen werden kann, was das Hantieren verzögert und erschwert.

Ferner zeigt EP 1 972 290 einen weiteren Träger für eine Knochenschraube. Die Knochenschraube ist mit Hilfe von zungenförmigen Halteelementen einklemmbar. Die dort dargestellten Träger erlauben jedoch ein Verkippen der Knochenschraube, was einer sicheren Lagerung im Wege steht. Darüber hinaus muss auch bei diesem Träger die Schraube zunächst quer zu ihrer Längsachse bewegt werden, damit sie aus dem Träger entnehmbar ist. Dies erfordert einerseits einen grösseren Platzbedarf, da die Knochenschraube zunächst seitlich bewegt werden muss. Weiterhin ist das für das Entnehmen erforderliche Hantieren recht mühsam.

Einige Knochenschrauben weisen einen Schaft und eine daran angeordnete Querschnittsverdickung auf. Als Beispiel seien so genannte intermaxillare Fixationsschrauben (IMF-Schrauben) erwähnt, welche an sich bekannt sind. Die Querschnittsverdickung kann dabei einen Durchmesser aufweisen, der nur geringfügig kleiner oder sogar grösser sein kann als der Durchmesser des Schraubenkopfes. Die oben beschriebenen, aus dem Stand der Technik bekannten Träger sind für die Aufnahme und Lagerung solcher Schrauben nicht geeignet. Beispielsweise würde bei einem wie in WO 2005/092231 dargestellten Träger die Knochenschraube mit der Unterseite der Querschnittsverdickung auf der Oberseite der Lagerfläche des Trägers aufliegen. Der Schraubenkopf würde folglich weit über die Lagerfläche hinausragen, so dass die Abdeckung noch grösser dimensioniert werden müsste.

Aus der US 2008/0016989 A1 ist ein Schraubendreher mit einer automatischen Zuführung bekannt. Der Schraubendreher enthält ein Band, an dem mehrere Schrauben befestigt sind. Die Schrauben werden durch einen Reibschluss oder durch Projektionen gehalten.

Es ist eine Aufgabe der vorliegenden Erfindung, die Nachteile des Bekannten zu überwinden und insbesondere einen Träger zur Aufnahme mindestens eines chirurgischen Gegenstandes bereitzustellen, in welchem der Gegenstand stabil gehalten werden kann und aus dem er leicht entnehmbar ist. Darüber hinaus soll die Baugrösse des Trägers möglichst gering gehalten werden.

Ein erster, aber nicht in der vollen hier beschriebenen Breite zur Erfindung gehöriger Aspekt betrifft einen Träger zur Aufnahme mindestens eines länglichen chirurgischen Gegenstandes, welcher einen Schaft und eine am Schaft angeordnete Querschnittsverdickung aufweist. Bei dem länglichen chirurgischen Gegenstand kann es sich beispielsweise um eine Knochenschraube oder einen Knochennagel handeln. Der Träger weist Haltemittel zum Halten des chirurgischen Gegenstandes im Bereich der Querschnittsverdickung seines Schaftes auf. Insbesondere können die Haltemittel zum Abstützen und/oder Klemmen des chirurgischen Gegenstandes im Bereich der Querschnittsverdickung ausgebildet sein.

Aufgrund des Haltens im Bereich der Querschnittsverdickung kann der chirurgische Gegenstand stabiler vom Träger gehalten werden. Da der chirurgische Gegenstand in einem Bereich seines Schaftes gehalten wird, in dem sein Querschnitt ohnehin grösser ist, können die möglichen Kontaktflächen zwischen den Haltemitteln und dem chirurgischen Gegenstand recht gross gewählt werden, was die Stabilität verstärkt. Darüber hinaus führt ein Halten im Bereich der Querschnittsverdickung dazu, dass ein Kopf des chirurgischen Gegenstandes, beispielsweise ein Schraubenknopf einer Knochenschraube oder ein Nagelkopf eines Knochennagels, nur wenig über diese Haltemittel hinausragen muss, was die Gesamtbauhöhe verringert.

Bevorzugt umfassen die Haltemittel mindestens eine Haltefläche, wobei der chirurgische Gegenstand durch Kontakt der Haltefläche mit der Querschnittsverdickung haltbar ist. Da somit der chirurgische Gegenstand in einem Bereich seines Schaftes gehalten wird, in dem sein Querschnitt ohnehin grösser ist, kann auch diese Haltefläche derart gross sein, dass ein besonders stabiles Halten möglich ist.

Bevorzugt enthält der Träger mindestens einen Haltevorsprung, welcher die Haltefläche aufweist. Der Haltevorsprung kann sich bezogen auf eine Achse einer wie unten beschrieben Aufnahmeöffnung des Trägers radial nach innen erstrecken. Alternativ kann er sich parallel zu dieser Achse erstrecken.

In einigen Ausführungsformen kann der Haltevorsprung eine insbesondere abgerundete und/oder abgeschrägte Einführfläche aufweisen, welche derart ausgebildet und angeordnet ist, dass der Haltevorsprung beim Einführen des Gegenstandes in den Träger aufgrund eines Kontaktes des Gegenstandes mit der Einführfläche bewegbar ist. Insbesondere kann hierdurch der Haltevorsprung im Wesentlichen senkrecht zu einer Einführrichtung bewegt werden, in welcher der Gegenstand in den Träger eingeführt wird. Dies ermöglicht ein einfaches Einführen des Gegenstandes in den Träger, da sich der Haltevorsprung beim Einführen von selbst in eine Position bewegt, in welcher er das Einführen nicht behindert. Ausserdem kann eine Abrundung oder Abschrägung des Haltevorsprungs eine Beschädigung eines Gewindes an einem Schraubenschaft verhindern, die ansonsten beim Einführen der Knochenschraube entstehen könnte.

Vorteilhafterweise ist die mindestens eine Haltefläche derart angeordnet, dass die Querschnittsverdickung eines vom Träger aufgenommenen chirurgischen Gegenstandes durch eine in radialer Richtung auf die Querschnittsverdickung wirkende Kraft haltbar ist. Bei dieser radialen Haltekraft kann es sich insbesondere um eine Klemmkraft handeln. Hierdurch wird der chirurgische Gegenstand besonders stabil gehalten. Alternativ kann der chirurgische Gegenstand durch die Haltemittel auch lediglich abgestützt werden, wodurch ein Verkippen der Schraubenachse verhindert werden kann.

In einigen möglichen Ausführungsformen können die Haltemittel mindestens zwei insbesondere voneinander beabstandete Halteflächen aufweisen, welche derart angeordnet sind, dass sie die Querschnittsverdickung eines vom Träger aufgenommenen chirurgischen Gegenstandes umschliessen. Hierdurch wird die Gefahr eines Verkippens einer Längsachse des chirurgischen Gegenstandes weiter reduziert. Die Längsachse kann beispielsweise durch die Schraubenachse einer Knochenschraube definiert sein. Falls die Halteflächen voneinander beabstandet sind, kann hierdurch zumindest ein Teil der Querschnittsverdickung kontaktfrei bleiben. Dies erleichtert die Passgenauigkeit zwischen den Haltemitteln und der Querschnittsverdickung. Überdies ist hierdurch ein grösserer Teil der Oberfläche des chirurgischen Gegenstandes einer Reinigung und Sterilisierung zugänglich.

Bevorzugt überdecken die Halteflächen einen Anteil im Bereich von 0,1 % bis 100 %, weiter bevorzugt im Bereich von 5 % bis 50 %, besonders bevorzugt im Bereich von 15 % bis 25 % des Umfangs der Querschnittsverdickung der Knochenschraube.

Vorteilhaft umfassen die Haltemittel alternativ oder zusätzlich eine Stützfläche, mittels welcher eine Unterseite der Querschnittsverdickung des chirurgischen Gegenstandes abstützbar ist. Mit der Unterseite der Querschnittsverdickung einer Knochenschraube ist dabei die dem Schraubenkopf abgewandte Seite der Querschnittsverdickung gemeint; mit der Unterseite der Querschnittsverdickung eines Knochennagels ist entsprechend die dem Nagelkopf abgewandte Seite der Querschnittsverdickung gemeint.

Ausserdem offenbart ist (ohne in der vollen hier beschriebenen Breite zur Erfindung zu gehören) eine Einheit aus einem wie oben beschriebenen Träger und mindestens einem länglichen chirurgischen Gegenstand, welcher einen Schaft und eine am Schaft angeordnete Querschnittsverdickung aufweist. Bei dem chirurgischen Gegenstand kann es sich beispielsweise um eine Knochenschraube oder einen Knochennagel handeln. Die Querschnittsverdickung ist von den Haltemitteln des Trägers gehalten, insbesondere eingeklemmt.

Ein erster Aspekt der Erfindung betrifft eine Einheit aus einem Träger zur Aufnahme mindestens eines chirurgischen Gegenstandes in einer Aufnahmeposition und mindestens einem chirurgischen Gegenstand. Der Träger kann eines, mehrere oder alle der oben beschriebenen Merkmale aufweisen. Bei dem Gegenstand handelt es sich um eine Knochenschraube oder einen Knochennagel. Auch die Knochenschraube oder der Knochennagel kann eines, mehrere oder alle der oben beschriebenen Merkmale aufweisen; beispielsweise kann es sich um eine Knochenschraube oder einen Knochennagel mit einer Querschnittsverdickung handeln. Der Träger umfasst einen Grundkörper und mindestens eine Stützfläche, welche derart ausgebildet und angeordnet ist, dass ein Stützbereich des Gegenstandes in der Aufnahmeposition in Kontakt mit der Stützfläche ist oder bringbar ist. Der Gegenstand ist in einer Entnahmerichtung aus der Aufnahmeposition entnehmbar. Die Entnahmerichtung verläuft dabei unter einem Winkel von höchstens 45°, bevorzugt höchstens 20°, besonders bevorzugt im Wesentlichen parallel zu einer Längsachse des in der Aufnahmeposition aufgenommenen Gegenstandes.

Erfindungsgemäss weist der Träger mindestens eine relativ zum Grundkörper bewegbare Haltefläche auf, welche derart ausgebildet und ausgeordnet ist, dass der Gegenstand in der Aufnahmeposition mittels der Haltefläche gehalten wird.

Dabei kann der Gegenstand insbesondere durch einen Kraftschluss haltbar sein, also etwa einklemmbar sein. Alternativ oder zusätzlich ist es auch möglich, dass der Gegenstand durch einen Formschluss haltbar ist. Beispielsweise kann die Haltefläche in den Zwischenraum zwischen zwei Gewindegängen einer Knochenschraube eindringen. Hierdurch kann eine Bewegung der Knochenschraube in deren Längsrichtung eingeschränkt werden.

Der Träger kann mindestens zwei relativ zum Grundkörper bewegbare Halteflächen aufweisen, zwischen denen der Gegenstand haltbar, insbesondere einklemmbar ist.

Alternativ oder zusätzlich kann der Träger eine relativ zum Grundkörper bewegbare Haltefläche mit mindestens zwei Halteflächenabschnitten enthalten, zwischen denen der Gegenstand haltbar, insbesondere einklemmbar ist. Dabei liegt es im Rahmen der Erfindung, dass die Haltefläche eine durchgehende Fläche ist und die Halteflächenabschnitte durch keine baulichen Strukturen, wie beispielsweise Nuten, voneinander getrennt sind; bei den Halteflächenabschnitten kann es sich also um rein gedanklich voneinander getrennte Abschnitte ein und derselben Fläche handeln.

Im Rahmen der vorliegenden Erfindung wird unter der Stützfläche auch eine Kante, also ein eindimensionaler Oberflächenbereich verstanden. Bevorzugt handelt es sich bei der Stützfläche jedoch um einen zweidimensionalen Oberflächenbereich.

Ebenso wird im Rahmen der vorliegenden Erfindung unter der Haltefläche auch eine Kante, also ein eindimensionaler Oberflächenbereich verstanden. Bevorzugt handelt es sich jedoch auch hierbei um einen zweidimensionalen Oberflächenbereich. Aufgrund der erfindungsgemässen Haltefläche ist es möglich, einen chirurgischen Gegenstand sicher in dem Träger zu halten. Da hierdurch zusätzliche Abdeckungen wie die in WO 2005/092231 gezeigte überflüssig werden, ergibt sich eine insgesamt geringere Baugrösse.

Mehrere der Träger mit darin aufgenommenen chirurgischen Gegenständen können nebeneinander, beispielsweise auf einer Bodenplatte eines chirurgischen Behälters angeordnet werden. Besonders bei länglichen Gegenständen, wie beispielsweise Knochenschrauben oder Knochennägeln, ist es zur Platzersparnis günstig, die Träger derart anzuordnen, dass die Längsachsen der Gegenstände in etwa senkrecht zur Bodenplatte ausgerichtet sind. Aufgrund des Winkels zwischen der Entnahmerichtung und der Längsachse sind die Gegenstände dann unter einem Winkel von höchstens 45 °, bevorzugt höchstens 20 ° in Bezug auf eine Senkrechte zur Bodenplatte oder besonders bevorzugt sogar im Wesentlichen senkrecht zur Bodenplatte entnehmbar. Die einzelnen Träger müssen also nicht einzeln aus dem Behälter entfernt werden, bevor der Gegenstand entnommen werden kann, wie dies etwa bei dem in WO 01/49198 gezeigten Träger der Fall ist.

Die Stützfläche des Trägers ist bei aufgenommenem chirurgie schem Gegenstand in Kontakt mit einem Stützbereich des Gegenstandes. Somit legt die Stützfläche die bestimmungsgemässe Aufnahmeposition des chirurgischen Gegenstandes relativ zum Träger fest. Der Gegenstand wird besonders gut durch die Stützfläche gestützt, wenn es sich hierbei um einen zweidimensionalen Oberflächenbereich handelt, also nicht lediglich um eine eindimensionale Kante. Mittels der Haltefläche oder Halteflächenbereiche wird der chirurgische Gegenstand in dieser Aufnahmeposition gehalten. Der Gegenstand wird besonders gut durch die Haltefläche oder die Halteflächenabschnitte gehalten, wenn es sich hierbei um zweidimensionale Oberflächenbereiche handelt, also nicht lediglich um eindimensionale Kanten. Selbstverständlich ist es denkbar und liegt im Rahmen der Erfindung, dass auch die Stützfläche ihrerseits den Gegenstand nicht nur stützt, sondern zusätzlich einklemmt.

Da die Haltefläche relativ zum Grundkörper bewegbar ist, handelt es sich um zwei voneinander verschiedene Flächen. Insbesondere können die Haltefläche und die Stützfläche räumlich voneinander getrennt sein. Daher wird auch der Gegenstand in zwei voneinander beabstandeten Bereichen gehalten, insbesondere eingeklemmt, bzw. gestützt, was zu einer besonders stabilen Lagerung führen kann.

Die Haltefläche ist mittels Federmitteln vorgespannt. Mittels dieser Federmittel kann beispielsweise eine Klemmwirkung erzielt werden. Alternativ ist es auch denkbar, dass die Federmittel die Haltefläche in einer derartigen Position relativ zum Gegenstand halten, in welcher die Haltefläche für einen Formschluss sorgt, indem sie beispielsweise in den Zwischenraum zwischen zwei Gewindegängen einer Knochenschraube eindringt. Die Federmittel umfassen mindestens eine Zunge, deren Ende die Haltefläche umfasst oder bildet. Insbesondere kann der Träger mehrere, beispielsweise drei Zungen umfassen, von denen jede an ihrem Ende eine Haltefläche umfasst oder bildet.

In der Aufnahmeposition erstreckt sich zumindest das Ende der Zunge in einer Zungenrichtung, welche einen spitzen Winkel mit der Entnahmerichtung bildet. Insbesondere kann diese Zungenrichtung im Wesentlichen mit der Entnahmerichtung übereinstimmen. Dies ermöglicht eine einfache Konstruktion und kann auch das Wiedereinsetzen des Gegenstandes in den Träger erschweren oder sogar vollständig verhindern (siehe unten).

In einigen Ausführungsformen ist die Haltefläche des Trägers derart ausgebildet und angeordnet, dass der Gegenstand in der Aufnahmeposition mittels der Haltefläche in einem Haltebereich haltbar ist, welcher im Bereich eines Schafts des Gegenstandes angeordnet ist. Insbesondere kann der Gegenstand im Haltebereich einklemmbar sein. Bei dem Gegenstand kann es sich beispielsweise um eine Knochenschraube oder um einen Knochennagel handeln. In diesem Haltebereich ist der Gegenstand, insbesondere die Knochenschraube oder der Knochennagel, besonders sicher haltbar, insbesondere einklemmbar.

Erfindungsgemäss ist die Stützfläche des Trägers derart ausgebildet und angeordnet, dass sie in der Aufnahmeposition mit der Unterseite einer Querschnittsverdickung des Gegenstandes in Kontakt bringbar ist. Dabei bildet die Unterseite den Stützbereich des Gegenstandes. Die Querschnittsverdickung kann insbesondere senkrecht zur Längsachse des Gegenstandes vorliegen. Bei der Querschnittsverdickung handelt es sich um den Kopf einer Knochenschraube oder eines Knochennagels. In diesem Stützbereich kann die Knochenschraube oder der Knochennagel besonders gut abgestützt werden. Alternativ kann beispielsweise die Querschnittsverdickung bei einer Knochenschraube mit zwei Gewindeabschnitten mit verschiedenen Durchmessern auch von dem Gewindeabschnitt mit grösserem Durchmesser gebildet werden.

Bevorzugt sind die Stützfläche und die Haltefläche in der Entnahmerichtung voneinander beabstandet. Hierdurch kann der Gegenstand besonders stabil gehalten und leicht entnommen werden.

Weiterhin bevorzugt ist der Gegenstand in der Aufnahmeposition mittels der Haltefläche in einer Klemmrichtung einklemmbar, die im Wesentlichen nicht parallel zur Entnahmerichtung ist. Insbesondere kann die Klemmrichtung im Wesentlichen senkrecht zur Entnahmerichtung sein. Auf diese Weise wird die Entnahme des Gegenstandes erleichtert.

Besonders bevorzugt weist der Träger mindestens zwei, bevorzugt mindestens drei, besonders bevorzugt genau drei Halteflächen auf. Weiterhin bevorzugt sind die Halteflächen im Wesentlichen gleichmässig um die Längsachse des in der Aufnahmeposition aufgenommenen Gegenstandes herum angeordnet. Im beispielhaften Fall von genau drei Halteflächen können diese Halteflächen also in einem Winkel von 120° in Umfangsrichtung um die Längsachse herum angeordnet sein. Dies garantiert ein besonders sicheres und einfaches Halten, insbesondere Einklemmen des Gegenstandes.

In einer möglichen Ausführungsform ist die Zunge einstückig mit dem Grundkörper verbunden. Insbesondere kann der gesamte Träger einstückig ausgebildet sein.

In einer weiteren, bevorzugten Ausführungsform enthält der Träger einen Einsatz, welcher einen Federring und mindestens eine der Zungen, bevorzugt alle Zungen umfasst. Der Federring ist dabei um die Längsachse des Gegenstandes herum angeordnet. Er kann beispielsweise in einer Aufnahmeöffnung des Trägers eingeklemmt oder eingeklebt sein, in welcher der Gegenstand zumindest teilweise aufgenommen ist. Der Federring kann geschlossen sein oder auch eine Durchbrechung aufweisen, welche das Einklemmen in eine korrespondierende Nut erleichtert. Die mindestens eine Zunge erstreckt sich von dem Federring.

In einigen Ausführungsformen ist zumindest ein Teil der Stützfläche ebenfalls am Ende der Zunge angeordnet. In anderen Ausführungsformen ist zumindest ein Teil der Stützfläche, insbesondere die gesamte Stützfläche am Grundkörper des Trägers angeordnet und kann von einem Teil der Oberfläche des Grundkörpers gebildet werden.

Besonders bevorzugt ist die Haltefläche derart ausgebildet und angeordnet, dass der Gegenstand in der Aufnahmeposition im Wesentlichen nicht in einer von der Entnahmerichtung verschiedenen Richtung bewegbar ist. Hierdurch kann verhindert werden, dass der Gegenstand versehentlich in eine derartige Position relativ zum Grundkörper des Trägers bewegt werden kann, aus welcher er nicht mehr entfernt werden kann.

Besonders bevorzugt ist die Haltefläche derart ausgebildet und angeordnet, dass der Gegenstand durch Ausübung einer ersten Kraft in der Entnahmerichtung bewegbar ist und in einer von der Entnahmerichtung verschiedenen, zweiten Richtung im Wesentlichen nicht bewegbar ist oder nur durch Ausübung einer zweiten Kraft bewegbar ist, welche grösser ist als die erste Kraft. Bei der zweiten Richtung kann es sich insbesondere um die der Entnahmerichtung entgegen gesetzte Richtung handeln. Aufgrund dieser Funktion kann der Gegenstand durch Ausübung einer gewissen Kraft aus dem Träger entnommen, jedoch nur mit grösserem Aufwand oder gar nicht mehr wieder in den Träger eingesetzt werden. Dieser Effekt kann beispielsweise dadurch erzielt werden, dass die Halteflächen an den Enden von Zungen angeordnet sind, wobei die Enden im Wesentlichen in die Entnahmerichtung gerichtet sind.

Besonders bevorzugt ist die Haltefläche in eine Sperrposition bringbar, insbesondere mittels der Federmittel, in welcher der Gegenstand nicht mehr im Träger aufnehmbar ist. Hierdurch kann verhindert werden, dass ein einmal entnommener Gegenstand wieder in den Träger eingesetzt werden kann. Ein versehentlich entnommener Gegenstand, der möglicherweise unsteril geworden ist, kann also nur erschwert wieder in den Träger eingesetzt werden und den falschen Anschein von Sterilität erwecken.

In einigen Ausführungsformen weist der Träger weiterhin Verbindungsmittel auf, mittels deren er mit einer Lagerungseinheit verbunden werden kann. Bei den Verbindungsmitteln kann es sich beispielsweise um Führungsvorsprünge handeln. Die Lagerungseinheit kann beispielsweise zwei Führungsschienen eines chirurgischen Behälters umfassen.

Der Grundkörper des Trägers kann beispielsweise einen biokompatiblen Kunststoff umfassen oder daraus bestehen, wie etwa PPSU oder PEEK. Er kann etwa durch Spritzgiessen oder durch Fräsen hergestellt werden. Alternativ kann er ein insbesondere biokompatibles Metall oder Nichtmetall umfassen oder daraus bestehen.

Der Grundkörper kann in der Längsachse eine Höhe von 5 mm bis 100 mm, bevorzugt von 5 mm bis 30 mm, besonders bevorzugt von 5 mm bis 15 mm aufweisen. Senkrecht zur Längsachse weist er eine Länge und eine Breite auf. Die Länge kann im Bereich von 3 mm bis 25 mm liegen. In einigen Ausführungsformen kann die Länge bevorzugt im Bereich von 10 mm bis 22 mm, besonders bevorzugt im Bereich von 15 mm bis 20 mm liegen. In anderen Ausführungsbeispielen kann die Länge im Bereich von 5 mm bis 20 mm, besonders bevorzugt von 8 mm bis 15 mm liegen. Die Breite kann im Bereich von 3 mm bis 25 mm, bevorzugt von 4 mm bis 15 mm, weiter bevorzugt von 5 mm bis 15 mm, besonders bevorzugt von 5 mm bis 10 mm und ganz besonders bevorzugt zwischen 6 mm und 10 mm liegen. Bevorzugt ist der Grundkörper im Wesentlichen quaderförmig ausgebildet.

Der Einsatz kann beispielsweise ein biokompatibles Metall umfassen oder daraus bestehen, welches für chirurgische Anwendungen geeignet ist, wie etwa Titan, einen rostfreien Federstahl oder eine Metalllegierung. Der Einsatz kann etwa durch Stanzen, Ätzen oder Lasern und anschliessendes Biegen hergestellt werden. Alternativ kann er einen insbesondere biokompatiblen Kunststoff enthalten oder daraus bestehen und etwa durch Fräsen oder Spritzgiessen hergestellt werden.

Ein weiterer Aspekt der Erfindung betrifft ein Osteosynthese-Set, welches mindestens einen Träger zur Aufnahme mindestens eines chirurgischen Gegenstandes, mindestens einen vom Träger in einer Aufnahmeposition gehaltenen chirurgischen Gegenstand und mindestens ein Entnahmewerkzeug zum Entnehmen des chirurgischen Gegenstandes aus dem Träger in einer Entnahmerichtung enthält. Bei dem Träger kann es sich insbesondere um einen wie oben beschriebenen Träger handeln. Der chirurgische Gegenstand ist in der Aufnahmeposition mit einer ersten Haltekraft vom Träger gehalten.

Das Entnahmewerkzeug ist durch Bewegung in einer der Entnahmerichtung entgegen gesetzten Richtung derart mit dem chirurgischen Gegenstand in Kontakt bringbar, dass in der Aufnahmeposition zwischen dem Entnahmewerkzeug und dem chirurgischen Gegenstand eine zweite Haltekraft wirkt, die grösser ist als die erste Haltekraft.

Aufgrund der hieraus resultierenden Kraft kann der chirurgische Gegenstand in der Entnahmerichtung aus dem Träger entnommen werden. Es genügt also allein die Bewegung des Entnahmewerkzeuges in der Entnahmerichtung, um diese erforderliche resultierende Kraft zu erzeugen. Der chirurgische Gegenstand ist somit besonders einfach aus dem Träger zu entnehmen.

Bevorzugt handelt es sich bei der ersten Haltekraft um eine erste Klemmkraft. Durch eine Klemmkraft ist der chirurgische Gegenstand besonders sicher vom Träger haltbar. Ausserdem stellen Mittel zum Erzeugen einer Klemmkraft nur geringe Anforderungen an die Passgenauigkeit.

In bevorzugten Ausführungsformen weist der Träger mindestens eine Haltefläche auf, welche in der Aufnahmeposition in Kontakt mit dem chirurgischen Gegenstand ist. Dieser Kontakt bewirkt dabei die erste Klemmkraft. Der Träger kann einen wie oben beschriebenen Haltevorsprung aufweisen, der die Haltefläche enthält.

Ebenfalls bevorzugt handelt es sich bei der zweiten Haltekraft um eine zweite Klemmkraft. Mit besonderem Vorteil ist das Entnahmewerkzeug selbst zum bestimmungsgemässen Handhaben des chirurgischen Gegenstandes ausgebildet. Der chirurgische Gegenstand kann also direkt mit dem Entnahmewerkzeug verwendet werden, beispielsweise während einer Operation.

In einigen Ausführungsformen handelt es sich bei dem chirurgischen Gegenstand um eine Knochenschraube, und bei dem Entnahmewerkzeug handelt es sich um einen Schraubendreher. Die zweite Haltekraft, insbesondere die zweite Klemmkraft, kann beispielsweise zwischen der Klinge des Schraubendrehers und einem Kopf der Knochenschraube wirken. Die Klinge des Schraubendrehers muss daher lediglich in eine Öffnung im Kopf der Knochenschraube eingeführt werden. Bei der Öffnung im Kopf kann es sich zum Beispiel um einen Schlitz, einen Kreuzschlitz, einen Torx, einen Inbus oder einen Vierkant handeln. Die Öffnung kann auch beispielsweise wie in DE 10 2004 026 769 oder in EP 1 987 792 beschrieben ausgebildet sein.

In einigen Ausführungsformen kann der chirurgische Gegenstand eine Knochenschraube sein, die einen Schaft und eine am Schaft angeordnete Querschnittsverdickung aufweist. Beispielsweise kann es sich um eine intermaxillare Fixationsschraube handeln. Die Knochenschraube kann im Bereich der Querschnittsverdickung ihres Schaftes von Haltemitteln des Trägers gehalten sein.

Schliesslich betrifft die Erfindung einen chirurgischen Behälter, der wenigstens eines der im Folgenden aufgeführten Elemente aufweist:
- mindestens eine wie oben beschriebene erfindungsgemässe Einheit;
- mindestens ein wie oben beschriebenes erfindungsgemässes Osteosynthese-Set.

Der chirurgische Behälter kann ausserdem wenigstens eines der im Folgenden aufgeführten Elemente aufweisen:
- mindestens einen wie oben beschriebenen nicht erfindungsgemässen Träger zur Aufnahme mindestens eines chirurgischen Gegenstandes;
- mindestens eine Einheit aus einem oben beschriebenen nicht erfindungsgemässen Träger und mindestens einem chirurgischen Gegenstand;

Im Folgenden wird die Erfindung anhand von Ausführungsbeispielen und Figuren näher erläutert. Dabei zeigen
- Figuren 1a und b:: eine erste Ausführungsform einer erfindungsgemässen Einheit aus einem Träger und einer darin aufgenommenen Knochenschraube in einer perspektivischen Ansicht bzw. einer seitlichen Schnittansicht;
- Figur 2:: eine Detailansicht eines Einsatzes des Trägers gemäss Figuren 1a und b in einer perspektivischen Ansicht;
- Figur 3:: den Träger gemäss Figuren 1a und b ohne Knochenschraube in einer seitlichen Schnittansicht;
- Figur 4:: eine zweite Ausführungsform einer erfindungsgemässen Einheit aus einem Trägers und einer darin aufgenommenen Knochenschraube in einer seitlichen Schnittansicht;
- Figuren 5a und b:: eine dritte Ausführungsform einer erfindungsgemässen Einheit aus einem Träger und einer darin aufgenommenen Knochenschraube in einer perspektivischen bzw. einer seitlichen Schnittansicht;
- Figur 6a:: eine Einheit aus einem Träger und einer Knochenschraube in einer perspektivischen Ansicht;
- Figur 6b:: den Trägers gemäss Figur 6a mit einer darin eingesetzten Knochenschraube in einer seitlichen Schnittansicht;
- Figur 7a:: einen Träger in einer perspektivischen Ansicht;
- Figur 7b:: den Träger gemäss Figur 7a mit einer darin eingesetzten Knochenschraube;
- Figur 8a:: einen Träger mit einer darin aufgenommenen Knochenschraube in einer perspektivischen Ansicht;
- Figur 8b:: den Träger gemäss Figur 8a ohne Knochenschraube in einer perspektivischen Ansicht;
- Figur 9a:: den Träger gemäss Figuren 8a und 8b mit darin aufgenommener Knochenschraube in einer Seitenansicht;
- Figur 9b:: den Träger gemäss Figuren 8a, 8b und 9a mit darin aufgenommener Knochenschraube und der Klinge eines Schraubendrehers in einer Seitenansicht;
- Figur 9c:: den Träger gemäss Figuren 8a, 8b, 9a und 9b und die mit Hilfe des Schraubendrehers aus dem Träger entnommene Knochenschraube in einer Seitenansicht;
- Figur 10:: einen chirurgischen Behälter mit mehreren Trägern mit jeweils einer Knochenschraube.

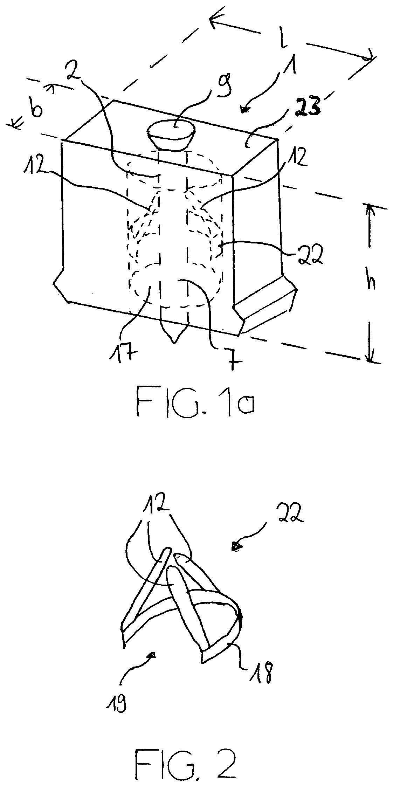

Die Figur 1a zeigt eine erste Ausführungsform eines Trägers 1 einer erfindungsgemässen Einheit mit einer darin aufgenommenen Knochenschraube 2 in einer perspektivischen Ansicht. Die Knochenschraube 2 bildet einen länglichen chirurgischen Gegenstand. Der Träger 1 umfasst einen Grundkörper 23, aus dem ein Kopf 9 der Knochenschraube 2 hinausragt. Ein Schaft 7 der Knochenschraube 2 ragt in eine Aufnahmeöffnung 17 des Grundkörpers 23 hinein, wo sie mit Hilfe von Zungen 12 eingeklemmt ist, welche Teil eines Einsatzes 22 sind (s. u.).

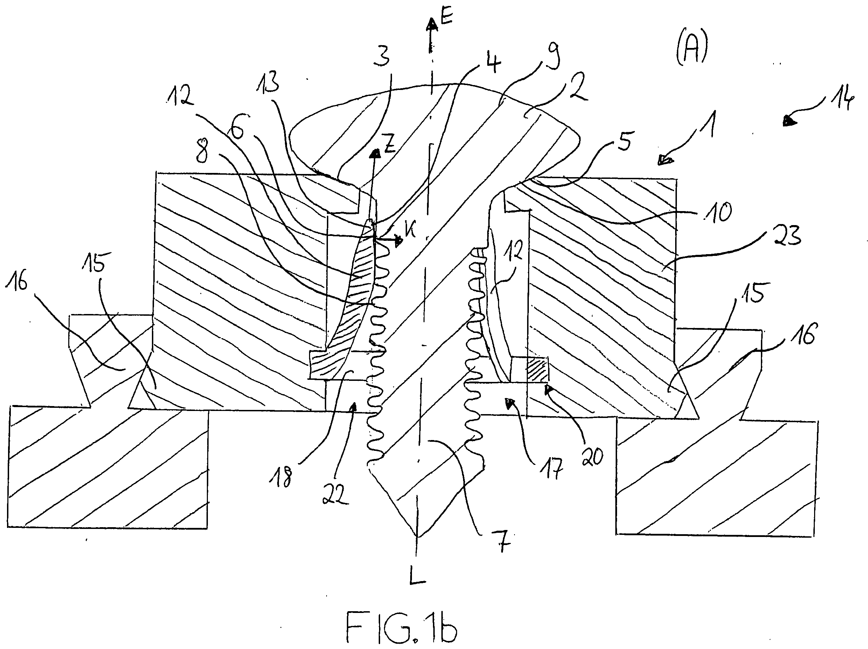

In Figur 1b ist der Träger 1 mit der darin aufgenommenen Knochenschraube 2 in einer seitlichen Schnittansicht dargestellt. Der Kopf 9 der Knochenschraube 2 bildet eine Querschnittsverdickung, dessen Unterseite 10 in Kontakt mit einer Stützfläche 3 des Trägers 1 ist. Ein Teil der Unterseite 10 des Kopfes 9 der Knochenschraube 2 bildet somit einen Stützbereich 5 der Knochenschraube 2. Die Stützfläche 3 gibt dabei die in Figur 1b dargestellte Aufnahmeposition A der Knochenschraube 2 in Bezug auf den Träger 1 vor.

Der Schaft 7 der Knochenschraube 2 ragt in die Aufnahmeöffnung 17 des Trägers 1 hinein. In eine umfängliche Nut 20 des Grundkörpers 23 des Trägers 1 ist ein Federring 18 eines Einsatzes 22 eingeklemmt (vgl. Detailansicht in Figur 2). Von dem Federring 18 erstrecken sich drei Zungen 12. Jede der drei Zungen 12 weist ein relativ zum Grundkörper 23 bewegbares Ende 13 auf, welches sich in einer Zungenrichtung Z erstreckt und jeweils eine Haltefläche 4 umfasst. Die Stützfläche 3 und die Halteflächen 4 sind in der Entnahmerichtung E voneinander beabstandet. Die Knochenschraube ist zwischen den Halteflächen 4 eingeklemmt und wird somit im Träger 1 gehalten. Die Halteflächen 4 sind gleichmässig um eine Längsachse L der Knochenschraube 2 angeordnet. Hierdurch wird die Knochenschraube 2 in einem Klemmbereich 6 in einer Klemmrichtung K eingeklemmt. Der Klemmbereich ist im Bereich des Gewindes 8 des Schafts 7 angeordnet. Die Klemmwirkung wird durch die Zungen 12 vermittelt, welche Federmittel bilden, mittels deren die Halteflächen 4 vorgespannt sind.

Die Knochenschraube 2 kann durch Ausübung einer ersten Kraft in einer Entnahmerichtung E entnommen werden. Der Träger 1 und insbesondere die Stützfläche 3 und die Halteflächen 4 sind dabei derart ausgebildet und angeordnet, dass die Knochenschraube 2 parallel zu ihrer Längsachse L entnehmbar ist. Die Zungenrichtung Z stimmt im Wesentlichen mit der Entnahmerichtung E überein. Dies hat zur Konsequenz, dass die Knochenschraube 2 im Wesentlichen nicht in einer der Entnahmerichtung E entgegengesetzten Richtung bewegbar ist oder nur durch Ausübung einer zweiten Kraft, welche grösser ist als die erste Kraft. Die Klemmrichtung K liegt senkrecht zur Entnahmerichtung E.

Der Träger 1 weist ferner Verbindungsmittel in Form von Führungsvorsprüngen 15 auf. Mit Hilfe dieser Führungsvorsprünge 15 ist der Träger 1 gemäss Figur 1b mit zwei Führungsschienen 16 verbunden, welche eine Lagerungseinheit für einen oder mehrere Träger 1 bildet. Entlang der Führungsschienen 16 ist der Träger 1 somit in einer zur Zeichenebene senkrechten Richtung verschiebbar.

Die Figur 2 zeigt eine Detailansicht des Einsatzes 22 des Trägers 1 gemäss den Figuren 1a und 1b. Dieser Einsatz 22 weist einen Federring 18 mit einer Durchbrechung 19 auf. Von dem Federring 18 erstrecken sich drei Zungen 12.

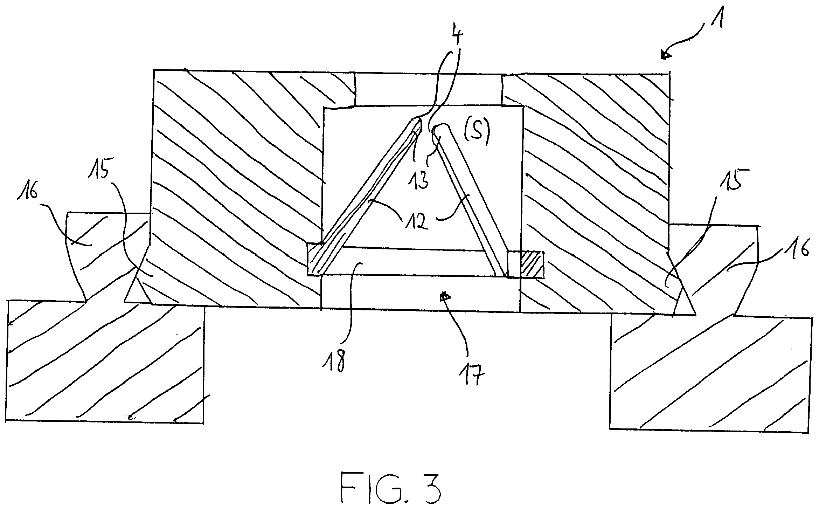

In Figur 3 ist der Träger 1 gemäss den Figuren 1a und 1b ohne Knochenschraube in einer seitlichen Schnittansicht dargestellt. Aufgrund der Vorspannung der Zungen 12 sind deren Enden 13 bei der Entnahme der Knochenschraube weiter in radialer Richtung in die Aufnahmeöffnung 17 eingedrungen. In dieser Position der Zungen 12 und der Halteflächen 4 ist die Knochenschraube nicht mehr in den Träger 1 einsetzbar. Im Sinne der Erfindung befinden sich die Halteflächen 4 damit in einer Sperrposition S. Eine versehentlich entnommene und möglicherweise unsteril gewordene Knochenschraube kann somit nicht wieder in den Träger 1 eingesetzt werden.

Der Grundkörper 23 des Trägers 1 kann beispielsweise aus einem biokompatiblen Kunststoff bestehen, wie etwa PPSU oder PEEK. Er kann etwa durch Spritzgiessen oder durch Fräsen hergestellt werden. Alternativ kann der Grundkörper 23 ein insbesondere biokompatibles Metall umfassen oder daraus bestehen. Mit Ausnahme der Führungsvorsprünge 15 ist der Grundkörper 23 im Wesentlichen quaderförmig und hat eine Länge 1 von 18 mm, eine Breite b von 8 mm und eine Höhe h von 8 mm.

Der Einsatz 22 kann beispielsweise aus einem biokompatiblen Metall bestehen, wie etwa Titan oder Stahl. Der Einsatz 22 kann etwa durch Stanzen, Ätzen oder Lasern und anschliessendes Biegen hergestellt werden. Alternativ kann er einen insbesondere biokompatiblen Kunststoff enthalten oder daraus bestehen und etwa durch Fräsen oder Spritzgiessen hergestellt werden.

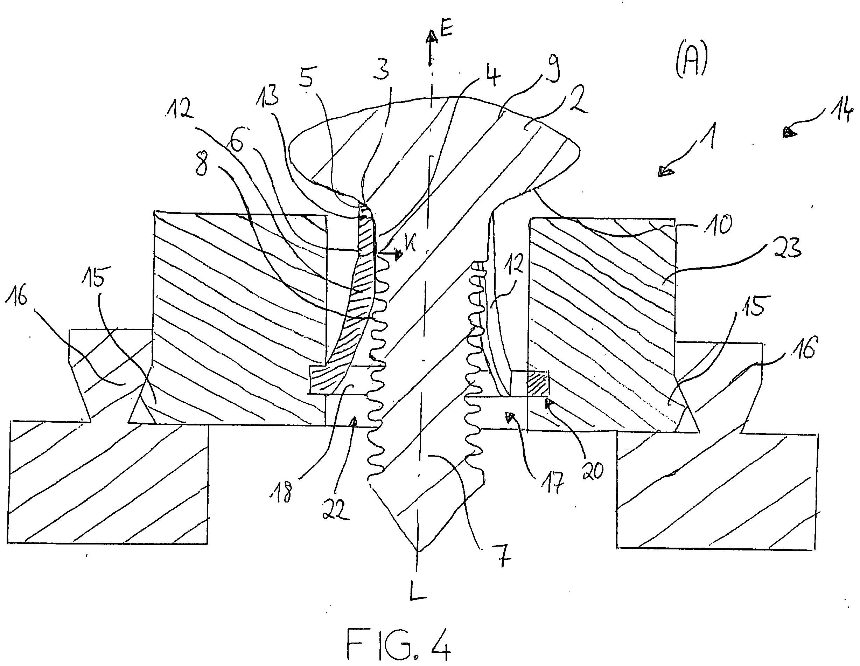

Die Figur 4a zeigt eine zweite Ausführungsform einer erfindungsgemässen Einheit 14 aus einem Trägers 1 und einer darin aufgenommenen Knochenschraube 2 in einer seitlichen Schnittansicht. Im Gegensatz zum ersten Ausführungsbeispiel sind hier die Stützflächen 3 nicht am Grundkörper 23 des Trägers 1 angeordnet, sondern ebenfalls an den Enden 13 von Zungen 12. Auch in diesem Ausführungsbeispiel sind die Stützflächen 3 in Kontakt mit einer Unterseite 10 eines Kopfes 9 der Knochenschraube 2. Eine solche Ausführungsform kann einfacher und kostengünstiger hergestellt werden.

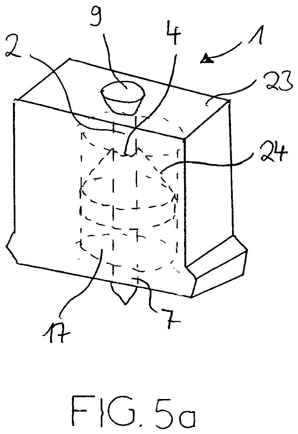

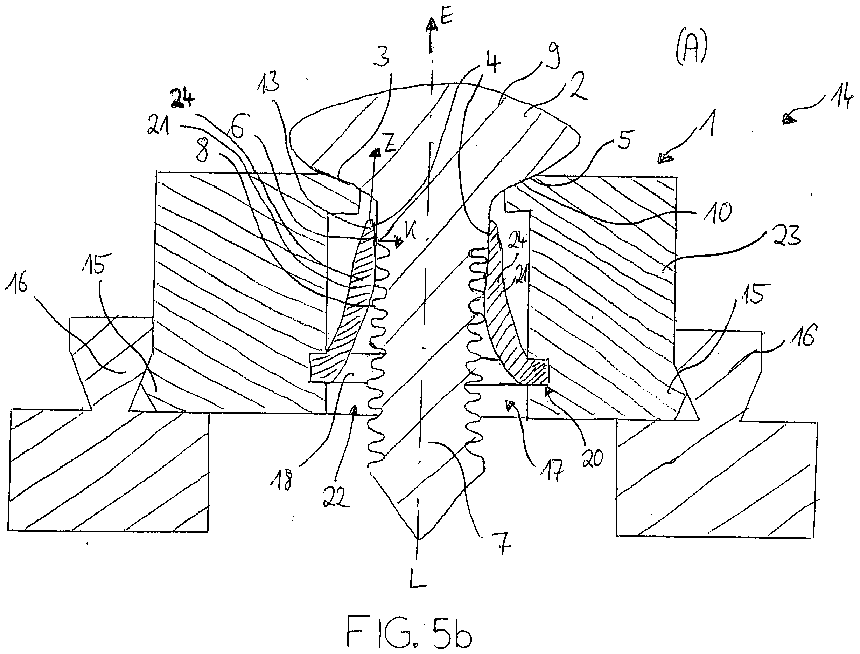

Ein drittes Ausführungsbeispiel einer erfindungsgemässen Einheit 14 aus einem Trägers 1 und einer aufgenommenen Knochenschraube 2 ist in den Figuren 5a und b dargestellt. Die Figur 5a zeigt den Träger 1 in einer perspektivischen Ansicht. Im Gegensatz zum ersten Ausführungsbeispiel gemäss Figuren 1a bis 3 enthält der Träger 1 keine Zungen, sondern einen Kranz 24, dessen kreisförmiger Rand eine einzige, durchgehende Haltefläche 4 bildet. Wie in der seitlichen Schnittansicht gemäss Figur 5b zu erkennen ist, ist der Schaft 7 der Knochenschraube 2 zwischen zwei Halteflächenabschnitten 21 der Haltefläche 4 eingeklemmt.

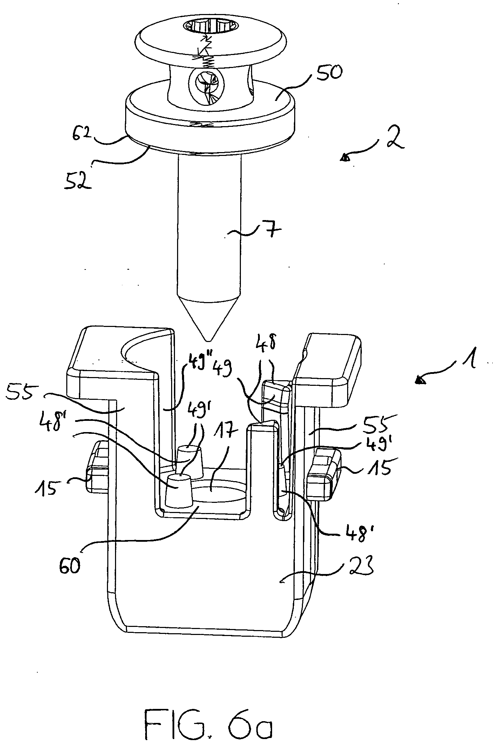

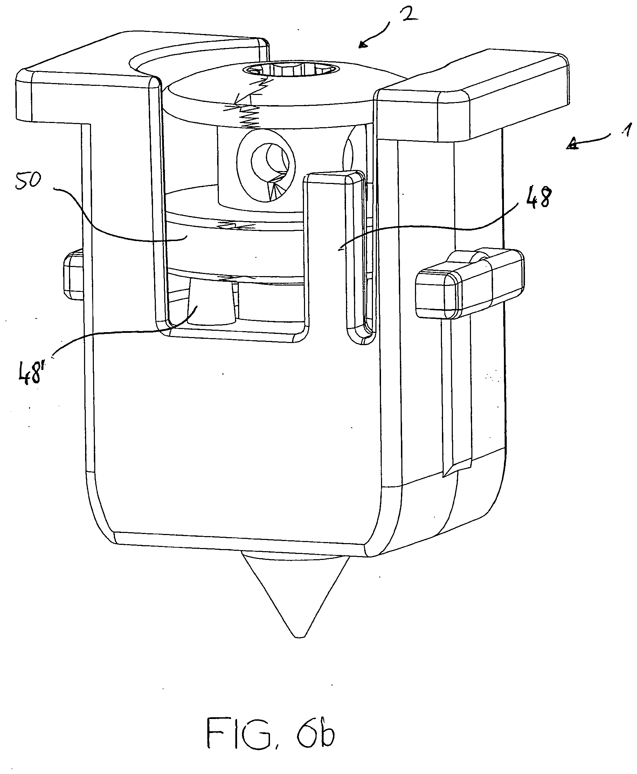

Die Figuren 6a und 6b zeigen eine weitere Einheit aus einem Träger 1 und einer Knochenschraube 2. Gemäss der perspektivischen Ansicht in Figur 6a weist der Träger 1 einen Grundkörper 23 mit zwei Seitenteilen 55 und einer Aufnahmeöffnung 17 für eine Knochenschraube 2 auf. Die Knochenschraube 2 weist einen Schaft 7 und eine am Schaft 7 angeordnete Querschnittsverdickung 50 auf. Bei dieser Knochenschraube 2 handelt es sich um eine intermaxillare Fixationsschraube. Der Träger 1 enthält Verbindungsmittel in Form von Führungsvorsprüngen 15. Mit Hilfe dieser Führungsvorsprünge 15 ist der Träger 1 analog zu Figur 4 entlang von Führungsschienen verschiebbar.

Der Träger 1 umfasst eine im Wesentlichen rechteckige Oberseite 60, welche senkrecht zur Aufnahmeöffnung 17 angeordnet ist. Der Träger 1 weist ferner fünf Haltevorsprünge 48,48' auf, von denen vier an jeweils einer der vier Ecken der Oberseite 60 angeordnet sind. Ein weiterer Haltevorsprung 48' ist zwischen zwei Ecken der Oberseite 60 und benachbart zu einem der Seitenteile 55 angeordnet. Die Haltevorsprünge 48,48' erstrecken sich senkrecht von der Oberseite 60 weg und damit parallel zur Aufnahmeöffnung 17. Zwei der Haltevorsprünge 48 weisen entlang ihrer Erstreckung parallel zur Aufnahmeöffnung 17 eine erste Länge auf, während die drei anderen Haltevorsprünge 48' kegelstumpfförmig ausgebildet sind entlang ihrer Erstreckung parallel zur Aufnahmeöffnung 17 eine zweite Länge aufweisen, die kleiner ist als die ersten Länge.

Die Haltevorsprünge 48,48' weisen jeweils eine Haltefläche 49,49' auf, mit welchen eine Knochenschraube 2 an einer Querschnittsverdickung 50 ihres Schaftes 7 haltbar ist (vgl. Figur 6b unten). Die Halteflächen 49 der Haltevorsprünge 48 mit der ersten Länge sind derart angeordnet, dass die Querschnittsverdickung 50 durch eine in radialer Richtung auf die Querschnittsverdickung 50 wirkende Haltekraft haltbar ist. Eine weitere Haltefläche 49" ist von einem Teil der Oberfläche des Seitenteiles 55 gebildet, welches den Haltvorsprüngen 48 mit der ersten Länge gegenüber liegt. Diese Oberfläche ist leicht konisch ausgebildet. Bei einer Querschnittsverdickung 50 mit zylindrischer Aussenseite entsteht dann ein linienförmiger Kontakt zwischen einem Teil der unteren Kante 62 der Querschnittsverdickung 50 und der Haltefläche 49" . Auf diese Weise wird die Querschnittsverdickung 50 von den drei Halteflächen 49,49" in radialer Richtung eingeklemmt.

Die Halteflächen 49' der Haltevorsprünge 48' mit der zweiten Länge sind derart angeordnet, dass die Querschnittsverdickung 50 durch eine in axialer Richtung auf die Querschnittsverdickung 50 wirkende Haltekraft haltbar ist. Hierdurch kann die Querschnittsverdickung 50 durch eine Dreipunktauflage in axialer Richtung gestützt werden.

Die fünf Halteflächen 49,49' sind voneinander beabstandet angeordnet, um die Zuführung von flüssigem oder gasförmigem Reinigung- und Sterilisationsmittel zu vereinfachen.

In Figur 6b ist eine weitere perspektivische Ansicht des Trägers 1 gemäss Figur 6a mit der darin eingesetzter Knochenschraube 2 dargestellt. Durch Kontakt der beiden Halteflächen 49 der Haltevorsprünge 48 mit der ersten Länge mit der Querschnittsverdickung 50 der Knochenschraube 2 ist die Knochenschraube 2 seitlich eingeklemmt. Hierdurch wird eine in radialer Richtung auf die Querschnittsverdickung 50 wirkende Haltekraft erzeugt, bei der es sich hier um eine Klemmkraft handelt. Die Unterseite 52 der Querschnittsverdickung 50 ist auf den hier nicht erkennbaren Halteflächen 49' der Haltevorsprünge 48' mit der zweiten Länge abgestützt.

Der Träger 1 gemäss Figuren 6a und 6b hat parallel zur Achse der Aufnahmeöffnung 17 eine Höhe von 9,6 mm, parallel zu den Führungsvorsprüngen 15 eine Weite von 5,9 mm und senkrecht zu diesen beiden Richtungen eine Tiefe von 9,8 mm.

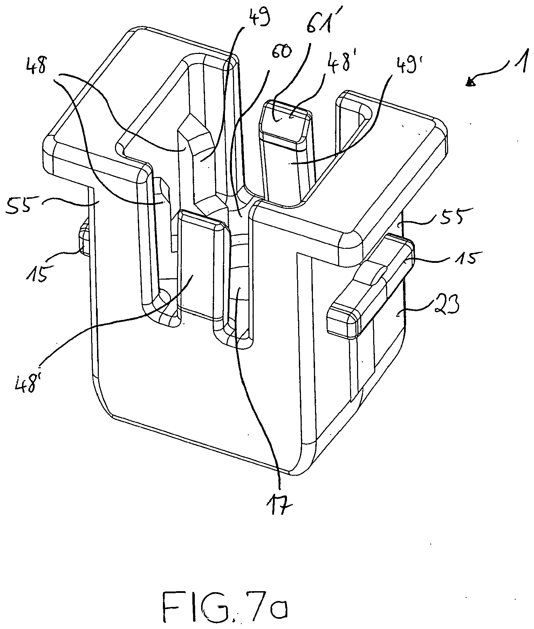

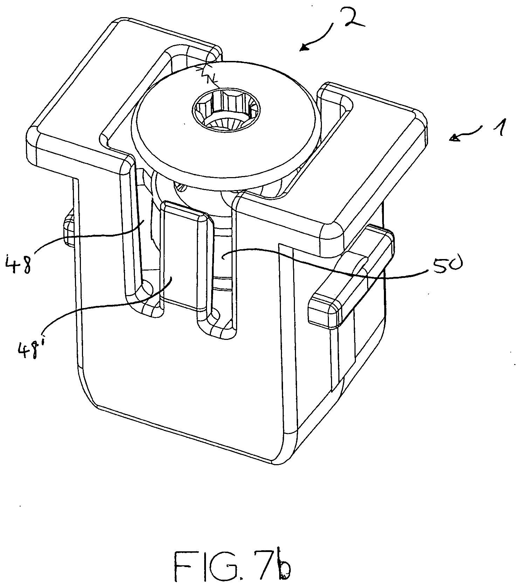

Weiterhin zeigen die Figuren 7a und 7b ein weiteres Ausführungsbeispiel eines Trägers 1. Dieser enthält gemäss Figur 7a ebenfalls einen Grundkörper 23, zwei Seitenteile 55, zwei Führungsvorsprünge 15, eine Oberseite 60 sowie eine sich dazu senkrecht erstreckende Aufnahmeöffnung 17 für eine hier nicht dargestellte Knochenschraube. Der Träger 1 dieses Ausführungsbeispieles enthält insgesamt sechs Haltevorsprünge 48, 48', von denen hier nur vier erkennbar sind. Jeweils zwei Haltevorsprünge 48 erstrecken sich von jedem der beiden Seitenteile 55. Die beiden weiteren Haltevorsprünge 48' erstrecken sich senkrecht von der Oberfläche 60 und damit parallel zur Aufnahmeöffnung 17.

Jeder der Haltevorsprünge 48,48' enthält eine Haltefläche 49,49', welche radial nach innen in Bezug auf die durch die Aufnahmeöffnung 17 definierte Achse gerichtet ist. Diese sechs Halteflächen 49,49' sind alle voneinander beabstandet und können die Querschnittsverdickung einer vom Träger aufgenommenen Knochenschraube umschliessen (vgl. Figur 7b unten). Auf diese Weise kann die eingesetzte Knochenschraube besonders sicher gehalten werden.

Am ihrem oberen Ende weisen die Haltevorsprünge 48' jeweils eine abgeschrägte Einführfläche 61' auf, welche zu den Halteflächen 49' benachbart sind. Diese Einführflächen 61' sind derart ausgebildet und angeordnet, dass die Haltevorsprünge 48' beim Einführen einer Knochenschraube in den Träger aufgrund eines Kontaktes des Gegenstandes mit den Einführflächen 61' radial nach aussen bewegbar sind. Dies ermöglicht ein einfaches Einführen der Knochenschraube in den Träger 1, da sich die Haltevorsprünge 48' beim Einführen aufgrund ihrer Flexibilität von selbst in eine Position bewegen, in welcher sie das Einführen nicht behindern.

Figur 7b zeigt den Träger 1 aus Figur 7a mit einer darin eingesetzten Knochenschraube 2 mit einer Querschnittsverdickung 50. Die hier nicht erkennbaren Halteflächen der Haltevorsprünge 48,48' halten die Knochenschraube 2 an ihrer Querschnittsverdickung 50 in radialer Richtung. Die Haltevorsprünge 48,48' sind elastisch ausgebildet, so dass sie die Knochenschraube 2 im Bereich ihrer Querschnittsverdickung 50 radial einklemmen.

Auch der Träger 1 gemäss den Figuren 7a und 7b hat parallel zur Achse der Aufnahmeöffnung 17 eine Höhe von 9,6 mm, parallel zu den Führungsvorsprüngen 15 eine Breite von 5,9 mm und senkrecht zu diesen beiden Richtungen eine Länge von 9,8 mm.

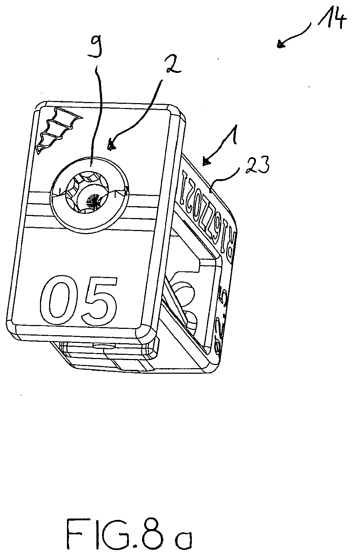

Eine weitere Ausführungsform eines Trägers 1 ist in den Figuren 8a bis 9c dargestellt. Gemäss der perspektivischen Ansicht in Figur 8a enthält der Träger 1 einen Grundkörper 23. In einer hier nicht erkennbaren Aufnahmeöffnung ist eine Knochenschraube 2 eingesetzt, von der hier lediglich der Kopf 9 sichtbar ist. Der Träger 1 und die Knochenschraube 2 bilden zusammen eine Einheit 14.

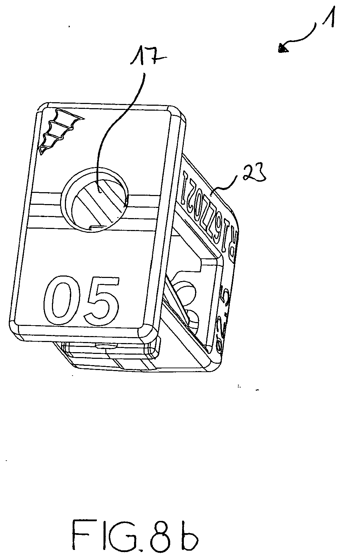

Figur 8b zeigt den gleichen Träger 1 in der gleichen perspektivischen Ansicht, allerdings ohne die eingesetzte Knochenschraube. In dieser Darstellung ist die Aufnahmeöffnung 17 für die Knochenschraube erkennbar.

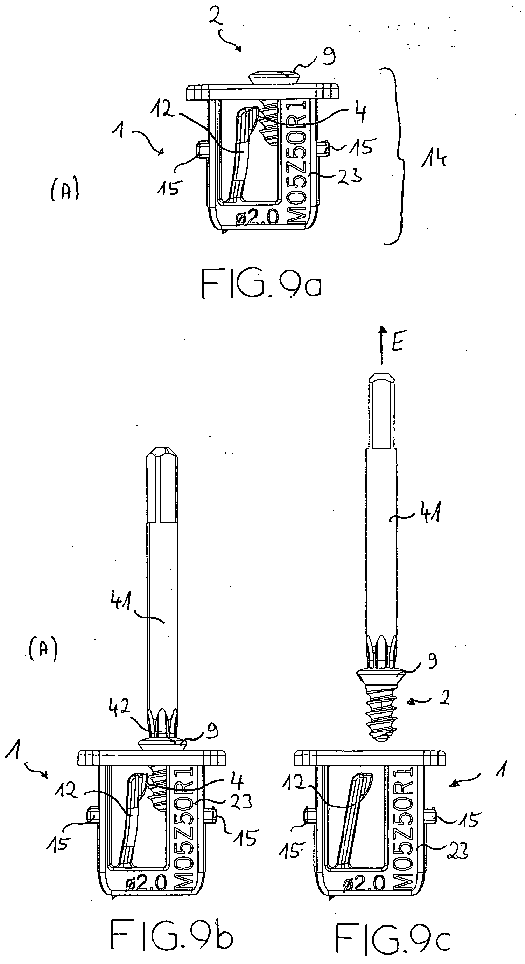

Figur 9a zeigt den Träger 1 gemäss Figuren 8a und 8b in einer seitlichen Ansicht. Der Träger 1 enthält an zwei gegenüberliegenden Seiten jeweils einen Führungsvorsprung 15. Mittels dieser Führungsvorsprünge 15 kann der Träger 1 analog zur Figur 4 entlang einer Führungsschiene verschoben werden. In der in Figur 9a dargestellten Aufnahmeposition A ist eine Knochenschraube 2 vom Träger 1 aufgenommen. Der Träger 1 weist eine Zunge 12 auf, an deren Ende eine Haltefläche 4 gebildet ist. Diese Haltefläche 4 ist in der dargestellten Aufnahmeposition A in Kontakt mit der Knochenschraube 2, wobei dieser Kontakt eine erste Klemmkraft bewirkt. Diese erste Klemmkraft bewirkt, dass die Knochenschraube 2 vom Träger 1 gehalten wird.

In Figur 9b ist die Einheit 14 aus Träger 1 und Knochenschraube 2 zusammen mit einem Schraubendreher 41 dargestellt. Die Einheit 14, welchen den Träger 1 und die Knochenschraube 2 umfasst, bildet zusammen mit dem Schraubendreher 41 ein Osteosynthese-Set. Der Schraubendreher 41 dient zum bestimmungsgemässen Handhaben der Knochenschraube 2 und wirkt gleichzeitig als Entnahmewerkzeug für die Knochenschraube 2.

Zur Erreichung der in Figur 9b dargestellten Position wurde der Schraubendreher 41 durch Bewegung nach unten derart mit der Knochenschraube 2 in Kontakt gebracht, dass zwischen der Klinge 42 des Schraubendrehers 41 und der Knochenschraube 2 eine zweite Haltekraft wirkt, die grösser ist als die erste Haltekraft. Bei der zweiten Haltekraft handelt es sich um eine zweite Klemmkraft, welche zwischen einer Klinge 42 des Schraubensziehers 41 und dem Kopf der Knochenschraube 2 wirkt.

Aufgrund der aus der ersten Klemmkraft und der zweiten Klemmkraft resultierenden Kraft kann die Knochenschraube 2 allein durch Bewegung des Schraubendrehers 41 in der Entnahmerichtung E vom Träger 1 entfernt werden, wie in Figur 9c dargestellt ist. Dies vereinfacht die Handhabung erheblich.

Die Träger 1 gemäss den Figuren 6a bis 9c können beispielsweise Kunststoff und/oder Metall enthalten oder daraus bestehen. Sie können etwa durch selektives Lasersintern, Fräsen, Vakuumgiessen oder Spritzgiessen hergestellt werden.

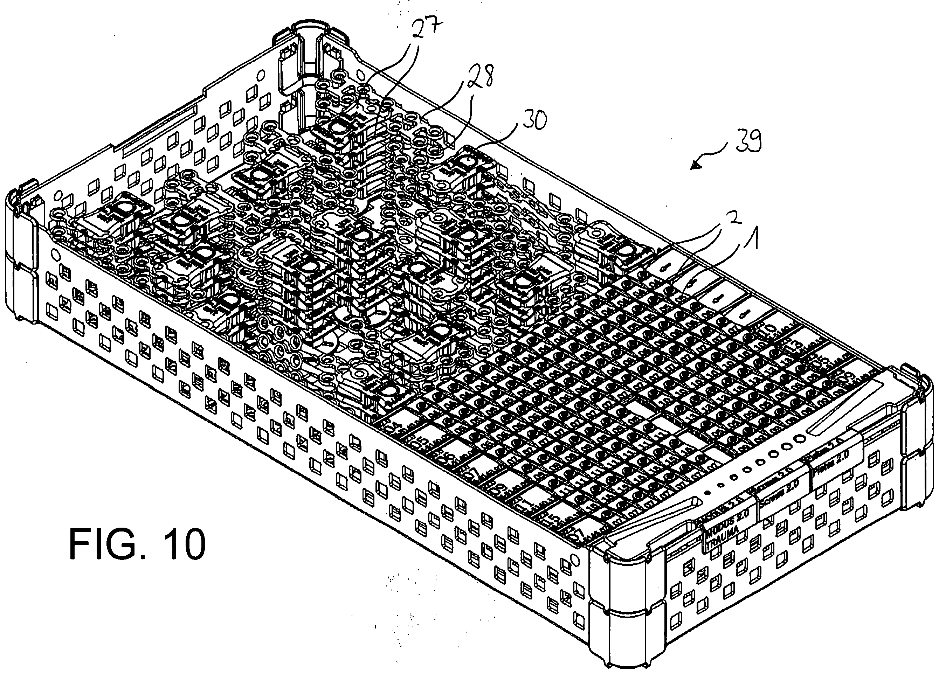

Die Figur 10 zeigt eine perspektivische Darstellung eines chirurgischen Behälters 39. Dieser chirurgische Behälter 39 enthält sowohl Halterungen mit Knochenplattenträgern 27 für Knochenplatten 28 als auch Träger 1 mit darin eingesetzten Knochenschrauben 2. Die Träger 1 sind entlang von Führungsschienen verschiebbar angeordnet.

## Patentansprüche

1. Einheit (14) aus
- einem Träger (1) zur Aufnahme mindestens eines chirurgischen Gegenstandes in einer Aufnahmeposition (A) und
- mindestens einem chirurgischen Gegenstand
wobei der Träger (1) umfasst:
- einen Grundkörper (23),
- mindestens eine relativ zum Grundkörper (23) bewegbare Haltefläche (4), welche derart ausgebildet und angeordnet ist, dass der Gegenstand in der Aufnahmeposition (A) mittels der Haltefläche (4) gehalten wird,
wobei
- der Gegenstand in einer Entnahmerichtung (E) aus der Aufnahmeposition (A) entnehmbar ist und
- die Entnahmerichtung (E) unter einem Winkel von höchstens 45° zu einer Längsachse (L) des in der Aufnahmeposition (A) aufgenommenen Gegenstandes verläuft,
- der Träger (1)
- mindestens zwei relativ zum Grundkörper (23) bewegbare Halteflächen (4) enthält, zwischen denen der Gegenstand haltbar ist und/oder
- mindestens eine relativ zum Grundkörper (23) bewegbare Haltefläche (4) mit mindestens zwei Halteflächenabschnitten(21) enthält, zwischen denen der Gegenstand haltbar ist,
- die Haltefläche (4) mittels Federmitteln vorgespannt ist und
- die Federmittel mindestens eine Zunge (12) umfassen, deren Ende (13) die Haltefläche (4) umfasst oder bildet,
wobei sich in der Aufnahmeposition (A) zumindest das Ende (13) der Zunge (12) in einer Zungenrichtung (Z) erstreckt, welche einen spitzen Winkel mit der Entnahmerichtung (E) bildet,
wobei der Träger (1) mindestens eine Stützfläche (3) umfasst, welche derart ausgebildet und angeordnet ist, dass ein Stützbereich (5) des Gegenstandes in der Aufnahmeposition (A) in Kontakt mit der Stützfläche (3) ist oder bringbar ist,
**dadurch gekennzeichnet,**
**dass** die Stützfläche (3) des Trägers (1) derart ausgebildet und angeordnet ist, dass sie in der Aufnahmeposition (A) mit der Unterseite (10) einer Querschnittsverdickung des Gegenstandes in Kontakt ist oder bringbar ist, wobei die Unterseite (10) den Stützbereich (5) bildet, wobei der Gegenstand als Knochenschraube (2) mit einem Kopf (9) oder als Knochennagel mit einem Kopf (9) ausgebildet ist, wobei der Kopf (9) die Querschnittsverdickung bildet.

2. Einheit (14) gemäss Anspruch 1, **dadurch gekennzeichnet, dass** die Haltefläche (4) des Trägers (1) derart ausgebildet und angeordnet ist, dass der Gegenstand in der Aufnahmeposition (A) mittels der Haltefläche (4) in einem Haltebereich (6) haltbar ist, welcher im Bereich eines Schafts (7) des Gegenstandes angeordnet ist.

3. Einheit (14) gemäss einem der Ansprüche 1 und 2,
**dadurch gekennzeichnet, dass**
die Stützfläche (3) und die Haltefläche (4) in der Entnahmerichtung (E) voneinander beabstandet sind.

4. Einheit (14) gemäss einem der Ansprüche 1 bis 3,
**dadurch gekennzeichnet, dass**
der Gegenstand in der Aufnahmeposition (A) mittels der Haltefläche (4) in einer Klemmrichtung (K) einklemmbar ist, die im Wesentlichen nicht parallel zur Entnahmerichtung (E).

5. Einheit (14) gemäss einem der Ansprüche 1 bis 4,
**dadurch gekennzeichnet, dass**
der Träger (1) mindestens zwei Halteflächen (4) aufweist.

6. Einheit (14) gemäss einem der Ansprüche 1 bis 5,
**dadurch gekennzeichnet, dass**
der Träger (1) einen Einsatz (22) enthält, welcher umfasst:
- einen Federring (18), welcher um die Längsachse (A) herum angeordnet ist,
- mindestens eine der Zungen (12), welche sich von dem Federring (18) erstrecken.

7. Einheit (14) gemäss einem der Ansprüche 1 bis 6,
**dadurch gekennzeichnet, dass**
zumindest ein Teil der Stützfläche (3) am Ende (13) der Zunge (12) angeordnet ist.

8. Einheit (14) gemäss einem der Ansprüche 1 bis 7,
**dadurch gekennzeichnet, dass**
zumindest ein Teil der Stützfläche (3) am Grundkörper (23) angeordnet ist.

9. Einheit (14) gemäss einem der Ansprüche 1 bis 8,
**dadurch gekennzeichnet, dass**
die Haltefläche (4) derart ausgebildet und angeordnet ist, dass der Gegenstand in der Aufnahmeposition (A) im Wesentlichen nicht in einer von der Entnahmerichtung (E) verschiedenen Richtung bewegbar ist.

10. Einheit (14) gemäss einem der Ansprüche 1 bis 9,
**dadurch gekennzeichnet, dass**
die Haltefläche (4) derart ausgebildet und angeordnet ist, dass der Gegenstand
- durch Ausübung einer ersten Kraft in der Entnahmerichtung (E) bewegbar ist und
- in einer von der Entnahmerichtung (E) verschiedenen,
- im Wesentlichen nicht bewegbar ist oder
- durch Ausübung einer zweiten Kraft bewegbar ist, welche grösser ist als die erste Kraft.

11. Einheit (14) gemäss einem der Ansprüche 1 bis 10, **gekennzeichnet durch** Verbindungsmittel zum Verbinden des Trägers (1) mit mindestens einer Lagerungseinheit (16).

12. Osteosynthese-Set, enthaltend
- mindestens eine Einheit (14) gemäss einem der vorangehenden Ansprüche, welche mindestens einen Träger (1) zur Aufnahme eines chirurgischen Gegenstandes, sowie mindestens einen vom Träger (1) in einer Aufnahmeposition (A) gehaltenen chirurgischen Gegenstand enthält,
- mindestens ein Entnahmewerkzeug zum Entnehmen des chirurgischen Gegenstandes aus dem Träger (1) in einer Entnahmerichtung (E),
wobei der chirurgische Gegenstand in der Aufnahmeposition (A) mit einer ersten Haltekraft vom Träger (1) gehalten ist,
**dadurch gekennzeichnet, dass**
das Entnahmewerkzeug durch Bewegung in einer der Entnahmerichtung (E) entgegengesetzten Richtung derart mit dem chirurgischen Gegenstand in Kontakt bringbar ist, dass in der Aufnahmeposition (A) zwischen dem Entnahmewerkzeug und dem chirurgischen Gegenstand eine zweite Haltekraft wirkt, die grösser ist als die erste Haltekraft.

13. Chirurgischer Behälter (39) enthaltend
- mindestens eine Einheit (33) gemäss einem der Ansprüche 1 bis 11 und/oder
- mindestens ein Osteosynthese-Set gemäss Anspruch 12.

## Claims

1. Unit (14) composed of
- a support (1) for receiving at least one surgical object in a receiving position (A) and
- at least one surgical object,
wherein said support (1) comprises
- a main body (23),
- at least one holding surface (4) which is movable relative to the main body (23) and which is designed and arranged in such a way that the object is held in the receiving position (A) by means of the holding surface (4),
wherein
- the object can be removed from the receiving position (A) in a removal direction (E), and
- the removal direction (E) extends at an angle of at most 45° to a longitudinal axis (L) of the object received in the receiving position (A),
- the support (1)
- has at least two holding surfaces (4) which are movable relative to the main body (23) and between which the object can be held
and/or
- has at least one holding surface (4) which is movable relative to the main body (23) and has at least two holding surface portions (21) between which the object can be held,
- the holding surface (4) is pretensioned by spring means and
- the spring means comprise at least one tongue (12) whose end comprises or forms the holding surface (4),
wherein in the receiving position (A), at least the end (13) of the tongue (12) extends in a tongue direction (Z) which forms an acute angle with the removal direction (E),
wherein the support (1) comprises at least one supporting surface (3) which is designed and arranged in such a way that a supporting area (5) of the object is or can be brought into contact with the supporting surface (3) in the receiving position (A),
**characterized in that**
the supporting surface (3) of the support (1) is designed and arranged such that in the receiving position (A), it is in contact with the underside (10) of a cross-sectional enlargement of the object, wherein the underside (10) forms the supporting area (5),
wherein the object is designed as a bone screw (2) with a head (9) or as a bone nail with a head (9), which head (9) forms the cross-sectional enlargement.

2. Unit (14) as claimed in claim 1, **characterized in that** the holding surface (4) of the support (1) is designed and arranged in such a way that the object in the receiving position (A) can be held by means of the holding surface (4) in a holding area (6) that is arranged in the area of a shank (7) of the object.

3. Unit (14) as claimed in one of claims 1 and 2, **characterized in that** the supporting surface (3) and the holding surface (4) are at a distance from each other in the removal direction (E).

4. Unit (14) as claimed in one of claims 1 through 3, **characterized in that** in the receiving position (A), the object can be clamped by means of the holding surface (4) in a clamping direction (K) which is substantially non-parallel to the removal direction (E).

5. Unit (14) as claimed in one of claims 1 through 4, **characterized in that** the support (1) has at least two holding surfaces (4).

6. Unit (14) as claimed in one of claims 1 through 5, **characterized in that** the support (1) contains an insert (22) which comprises:
- a spring ring (18) which is arranged around the longitudinal axis (A),
- at least one of the tongues (12), which tongue extends from the spring ring (18).

7. Unit (14) as claimed in one of claims 1 through 6, **characterized in that** at least part of the supporting surface (3) is arranged at the end (13) of the tongue (12).

8. Unit (14) as claimed in one of claims 1 through 7, **characterized in that** at least part of the supporting surface (3) is arranged on the main body (23).

9. Unit (14) as claimed in one of claims 1 through 8, **characterized in that** the holding surface (4) is designed and arranged in such a way that the object, in the receiving position (A), is substantially immovable in a direction different than the removal direction (E).

10. Unit (14) as claimed in one of claims 1 through 9, **characterized in that** the holding surface (4) is designed and arranged in such a way that the object
- is movable in the removal direction (E) by application of a first force, and,
- in a second direction different than the removal direction (E),
- is substantially immovable or
- is movable by application of a second force that is greater than the first force.

11. Unit (14) as claimed in one of claims 1 through 10, **characterized by** connecting means for connecting the support (1) to at least one storage unit (16).

12. Osteosynthesis set comprising
- at least one unit (14) as claimed in one of the preceding claims, comprising a support (1) for receiving a surgical object as well as at least one surgical object held by the support (1) in a receiving position (A),
- at least one removal tool for removing the surgical object from the support (1) in a removal direction (E),
wherein the surgical object, in the receiving position (A), is held by the support (1) with a first holding force,
**characterized in that**
the removal tool, by being moved in a direction counter to the removal direction (E), can be brought into contact with the surgical object in such a way that, in the receiving position (A), a second holding force acts between the removal tool and the surgical object, which second holding force is greater than the first holding force.

13. Surgical container (39) containing
- at least one unit (33) as claimed in one of claims 1 through 11 and/or
- at least one osteosynthesis set as claimed in claim 12.

## Revendications

1. Unité (14) composée de
- un support (1) destiné à recevoir au moins un objet chirurgical dans une position de réception (A) et
- au moins un objet chirurgical,
dans lequel le support (1) comprend
- un corps de base (23),
- au moins une face de maintien (4) mobile par rapport au corps de base (23), qui est configurée et disposée de telle manière que l'objet soit maintenu dans la position de réception (A) au moyen de la face de maintien (4),
dans lequel
- l'objet peut être enlevé hors de la position de réception (A) dans une direction d'enlèvement (E) et
- la direction d'enlèvement (E) s'étend sous un angle d'au plus 45° par rapport à un axe longitudinal (L) de l'objet placé dans la position de réception (A),
- le support (1)
- contient au moins deux faces de maintien (4) mobiles par rapport au corps de base (23), entre lesquelles l'objet peut être maintenu,
et/ou
- contient au moins une face de maintien (4) mobile par rapport au corps de base (23) avec au moins deux parties de face de maintien (21), entre lesquelles l'objet peut être maintenu,
- la face de maintien (4) est précontrainte au moyen de moyens de ressort et
- les moyens de ressort comprennent au moins une languette (12), dont l'extrémité (13) comprend ou forme la face de maintien (4),
dans lequel dans la position de réception (A) au moins l'extrémité (13) de la languette (12) s'étend dans une direction de languette (Z), qui forme un angle aigu avec la direction d'enlèvement (E),
dans lequel le support (1) comprend au moins une face d'appui (3), qui est configurée et disposée de telle manière qu'une région d'appui (5) de l'objet se trouve ou puisse être mise en contact avec la face d'appui (3) dans la position de réception (A),
**caractérisée en ce que** la face d'appui (3) du support (1) est configurée et disposée de telle manière qu'elle soit ou qu'elle puisse être mise en contact avec le côté inférieur (10) d'une surépaisseur de section transversale de l'objet dans la position de réception (A), dans lequel le côté inférieur (10) forme la région d'appui (5), dans lequel l'objet a la forme d'une vis d'os (2) avec une tête (9) ou un clou d'os avec une tête (9), dans lequel la tête (9) forme la surépaisseur de section transversale.

2. Unité (14) selon la revendication 1, **caractérisée en ce que** la face de maintien (4) du support (1) est configurée et disposée de telle manière que l'objet puisse être maintenu dans la position de réception (A) au moyen de la face de maintien (4) dans une région de maintien (6), qui est disposée dans la région d'une tige (7) de l'objet.

3. Unité (14) selon une des revendications 1 et 2, **caractérisée en ce que** la face d'appui (3) et la face de maintien (4) sont espacées l'une de l'autre dans la direction d'enlèvement (E).

4. Unité (14) selon l'une quelconque des revendications 1 à 3, **caractérisée en ce que** l'objet peut être serré dans la position de réception (A) au moyen de la face de maintien (4) dans une direction de serrage (K), qui n'est essentiellement pas parallèle à la direction d'enlèvement (E).

5. Unité (14) selon l'une quelconque des revendications 1 à 4, **caractérisée en ce que** le support (1) présente au moins deux faces de maintien (4).

6. Unité (14) selon l'une quelconque des revendications 1 à 5, **caractérisée en ce que** le support (1) contient un insert (22), qui comprend:
- une rondelle élastique (18), qui est placée autour de l'axe longitudinal (A),
- au moins une des languettes (12), qui s'étendent à partir de la rondelle élastique (18).

7. Unité (14) selon l'une quelconque des revendications 1 à 6, **caractérisée en ce qu'**au moins une partie de la face d'appui (3) est disposée à l'extrémité (13) de la languette (12).

8. Unité (14) selon l'une quelconque des revendications 1 à 7, **caractérisée en ce qu'**au moins une partie de la face d'appui (3) est disposée sur le corps de base (23).

9. Unité (14) selon l'une quelconque des revendications 1 à 8, **caractérisée en ce que** la face de maintien (4) est configurée et disposée de telle manière que l'objet dans la position de réception (A) ne soit essentiellement pas mobile dans une direction différente de la direction d'enlèvement (E).

10. Unité (14) selon l'une quelconque des revendications 1 à 9, **caractérisée en ce que** la face de maintien (4) est configurée et disposée de telle manière que l'objet
- soit mobile dans la direction d'enlèvement (E) par l'exercice d'une première force et
- dans une direction différente de la direction d'enlèvement (E):
- ne soit essentiellement pas mobile, ou
- soit mobile par l'exercice d'une deuxième force, qui est plus élevée que la première force.

11. Unité (14) selon l'une quelconque des revendications 1 à 10, **caractérisée par** des moyens d'assemblage pour assembler le support (1) à au moins une unité d'appui (16).

12. Ensemble d'ostéosynthèse, contenant
- au moins une unité (14) selon l'une quelconque des revendications précédentes, qui contient au moins un support (1) destiné à recevoir un objet chirurgical, ainsi qu'au moins un objet chirurgical maintenu par le support (1) dans une position de réception (A),
- au moins un outil d'enlèvement pour enlever l'objet chirurgical hors du support (1) dans une direction d'enlèvement (E),
dans lequel l'objet chirurgical est maintenu par le support (1) dans la position de réception (A) avec une première force de maintien,
**caractérisé en ce que** l'outil d'enlèvement peut être mis en contact avec l'objet chirurgical par un mouvement dans une direction opposée à la direction d'enlèvement (E), de telle manière que dans la position de réception (A) il agisse entre l'outil d'enlèvement et l'objet chirurgical une deuxième force de maintien, qui est plus grande que la première force de maintien.

13. Récipient chirurgical (39), contenant
- au moins une unité (33) selon l'une quelconque des revendications 1 à 11 et/ou
- au moins un ensemble d'ostéosynthèse selon la revendication 12.
